# EUROPEAN PATENT APPLICATION

(11) **EP 3 744 231 A1**
(43) Date of publication of application: **02.12.2020**
(21) Application number: 19741768.6
(22) Date of filing: 21.01.2019
(51) Int. Cl.: A61B 3/12, A61B 3/14

(54) **MULTISPECTRAL GENERATING UNIT, AND FUNDUS IMAGING SYSTEM AND METHOD**

(30) Priority: 22.01.2018 CN 201810066569
(71) Applicant: Shenzhen Thondar Technology Co., Ltd, Shenzhen, Guangdong 518000 (CN)
(72) Inventor: LIU, Kexing, Shenzhen, Guangdong 518000 (CN); DONG, Xuechuan, Shenzhen, Guangdong 518000 (CN); HUANG, Yequan, Shenzhen, Guangdong 518000 (CN); GONG, Hongwei, Shenzhen, Guangdong 518000 (CN); ZHANG, Xuelian, Shenzhen, Guangdong 518000 (CN); ZHANG, Jinsong, Shenzhen, Guangdong 518000 (CN); RIBARIC, Zeljko, Shenzhen, Guangdong 518000 (CN)
(74) Representative: Osterhoff, Utz
(86) International application number: PCT/CN2019/072569
(87) International publication number: WO 2019/141277

(57) **Abstract**

A multi-spectral light generating unit (100), a fundus imaging system, and a fundus imaging method. The multi-spectral light generating unit (100) includes one or more illumination units (102) and a control unit (101); each illumination unit (102) includes a light-emitting diode matrix, each light-emitting diode matrix emits light of multiple wavelengths; converting (S1502), by the control unit (101), the control instruction sent by the central controller (105) into a control signal; triggering (S1503), by the control unit (101), the specified light-emitting diode matrix of the specified illumination unit (102) to emit light of a preset wavelength and preset energy to output the multi-spectral light. The multi-spectral generating unit (100) may provides the multi-spectral generating unit with parameters meets the user's needs such as shape, wavelength, energy, and flash time, so as provide a multi-spectral generating unit with better performance for the fundus imaging system or other imaging and illumination systems, which improves imaging ability to make imaging clearer. In addition, the photoconductive device (103) may be set with different shapes and structures to output, so that the light of multi-spectral light has a set shape, such as full ring, half ring, quarter arc, etc. The central controller (105) may control other parameters of the multi-spectral light through the control unit (102), so that the final output of the multi-spectral light can meet user requirements in terms of shape, numerical aperture, and luminous area.

## Description

### CROSS-REFERENCE TO RELATED APPLICATIONS

This application claims the priority of Chinese Patent Application with No. 201810066569.6, entitled "MULTI-SPECTRAL LIGHT SOURCE, FUNDUS IMAGING SYSTEM AND METHOD", filed on January 22, 2018, which is hereby incorporated by reference in its entirety.

### FIELD

This application relates to the technical field of fundus imaging, in particular to a multi-spectral generating unit, a fundus imaging system, and a fundus imaging method.

### BACKGROUND

Generally, a halogen lamp or a xenon white light source, a light-emitting diode, or a scanning laser may be used as a light source of a fundus imaging system to obtain a fundus retinal image. Current pixel sensors of fundus cameras use different filter technologies to separate white light into red, green and blue spectra to obtain retinal images.

Color images of the retina or anterior segment of the eye may be generated by using xenon or other white light sources, and the retina imaging of the red, green, and blue image acquisition device, but the power of the xenon white light source illumination light at different wavelengths is quite different and has limitations. Separation of xenon white light into multiple narrow-band spectral filters with a large amount of tasks is difficult to achieve; at the same time, xenon light illumination is very strong, repeated irradiation will cause pupil contraction to reduce, making imaging difficult. Fundus imaging may also use laser scanning irradiation technology, the laser beam gives light through the pinhole. Few discrete wavelengths may be obtained by scanning lasers, but lasers are expensive, bulky, and require a large internal space of the device, so laser scanning is less practical and poses a great safety hazard to the use of lasers on the eyes.

In view of the above-mentioned problem that the performance of the light source configured for fundus imaging is poor, which results in the limitation of fundus imaging, no effective solution has been proposed.

### SUMMARY

In view of this, the purpose of this application is to provide a multi-spectral generating unit, a fundus imaging system, and a fundus imaging method, to provide a multi-spectral generating unit with better performance for the fundus imaging system or other imaging and illumination systems, thereby the imaging capability is improved, and the imaging is clearer.

In a first aspect, an embodiment of this application provides a multi-spectral light generating unit, which includes one or more illumination units and a control unit. The illumination unit includes a light-emitting diode matrix, each light-emitting diode matrix is configured to emit light of multiple wavelengths. The control unit is configured to convert a control instruction sent from a central controller into a control signal to trigger a specified light-emitting diode matrix of a specified illumination unit to emit light of a preset wavelength and preset energy to output multi-spectral light.

The multi-spectral light generating unit further includes a photoconductive device connected to the illumination unit.

The photoconductive device is configured to shape the light emitted from the light-emitting diode matrix of the illumination unit to output multi-spectral light of a set shape.

Preferably, the foregoing multi-spectral light generating unit further includes a beam shaping unit. An input end of the beam shaping unit is connected to the light-emitting diode matrix of the illumination unit, and an output end of the beam shaping unit is connected to the photoconductive device. The beam shaping unit is configured to adjust distribution of light of each wavelength emitted by the light-emitting diode matrix of the illumination unit to distribute the light of each wavelength uniformly on a cross section of the output end of the beam shaping unit. The photoconductive device is further configured to arrange optical fibers according to a set arrangement, and further shape output light of the beam shaping unit to output the multi-spectral light with the set shape.

Preferably, the multi-spectral light generating unit further includes a beam shaping unit, and an input end of the beam shaping unit is connected to the light-emitting diode matrix of the illumination unit. The beam shaping unit is configured to adjust distribution of light of each wavelength emitted by the light-emitting diode matrix of the illumination unit to distribute the light of each wavelength uniformly on a cross section of the output end of the beam shaping unit to output uniform multi-spectral light.

Preferably, the photoconductive device includes optical fiber sub-bundles matching a number of the light-emitting diode matrix of the illumination unit. Each optical fiber sub-bundle of the photoconductive devices includes a set number of optical fibers. At an input end of the photoconductive device, cores of each group of the optical fiber sub-bundles are uniformly arranged on a light-emitting surface of a corresponding light-emitting diode matrix. A position of each optical fiber sub-bundle is configured to correspond to a position of each light-emitting diode. Each optical fiber sub-bundle includes one or more optical fibers. Optical fibers with a set shape and a set number are formed at an output end of the photoconductive device.

Preferably, the above-mentioned beam shaping unit includes a light homogenizing device.

Preferably, the light homogenizing device is a light homogenizing rod. An input end of the light homogenizing rod is oppositely coupled to the light-emitting diode matrix of the illumination unit. A shape of an input end of the light homogenizing rod is configured to match a shape of the light-emitting diode matrix of the illumination unit. An output end of the light homogenizing rod is docked with an optical fiber bundle of the photoconductive device.

Preferably, the multi-spectral light generating unit further includes a beam uniform divergence unit mounted at an end of the photoconductive device to further spread out light of a light beam with a set shape uniformly.

Preferably, the above-mentioned beam uniform divergence unit is a frosted glass diffuser. The frosted glass diffuser has the characteristics of simplicity, low cost and good astigmatism.

Preferably, the light-emitting diode matrix of the above illumination unit is arranged in a square array.

Preferably, the multi-spectral light generating unit includes one or two illumination units, one or two beam shaping units corresponding to a number of the illumination units, and one or two photoconductive devices. The control unit is configured to convert the control instruction sent from the central controller into the control signal to control the one or two illumination units.

Preferably, an output end of each photoconductive device has a half-ring structure. The half-ring structures of the output ends of the two photoconductive devices are configured to match each other. The half-ring structures of the output ends of the two photoconductive devices are configured to be combined to form a full ring structure.

Preferably, the output end of the photoconductive device includes a full ring structure. The full ring structure is configured to arrange the optical fibers at the output end of the beam shaping unit into a full ring with a set radius. One full ring of the two output ends of the two photoconductive devices is an outer ring structure, and the other full ring of the two output ends of the two photoconductive devices is an inner ring structure, where a radius of the outer ring structure is larger than a radius of the inner ring structure.

Preferably, the output end of the photoconductive device includes two groups of quarter ring structures. Each quarter ring structure is configured to arrange the optical fibers at the output end of the photoconductive device in an arc shape. The quarter ring structures are uniformly distributed along a same circumference at the output ends of the two photoconductive devices.

In a second aspect, an embodiment of this application further provides a fundus imaging system including the above-mentioned multi-spectral light generating unit, and it also includes a central controller and an image acquisition device, and the central controller and the image acquisition device are in communication connection with the control unit, respectively.

The central controller is configured to issue a control instruction to the multi-spectral light generating unit.

The multi-spectral light generating unit is configured to convert a control instruction sent from a central controller into a control signal to trigger a specified light-emitting diode matrix of a specified illumination unit to emit light of a preset wavelength and preset energy to output multi-spectral light.

The image acquisition device is configured to collect a fundus image under an environment of the multi-spectral light.

In a third aspect, an embodiment of this application provides a multi-spectral light generating unit further including a light energy detector electrically connected to the control unit.

The light energy detector is mounted at one side of the light-emitting diode matrix, and configured to detect energy of the multi-spectral light and transmit the energy of the multi-spectral light to the control unit.

The control unit is configured to control the light-emitting diode of the illumination unit to turn off when the energy of the multi-spectral light is greater than a preset threshold.

In a fourth aspect, an embodiment of this application further provides a fundus imaging method, which is applied to a fundus imaging system including a central controller, an image acquisition device, and the above-described multi-spectral light generating unit. The central controller and the image acquisition device are in communication connection with the control unit, respectively, where the method includes:
issuing, by the central controller, a control instruction to the control unit;
converting, by the control unit, the control instruction sent by the central controller into a control signal;
triggering, by the control unit, the specified light-emitting diode matrix of the specified illumination unit to emit light of a preset wavelength and preset energy;
shaping, by the photoconductive device, the light emitted from the light-emitting diode matrix of the illumination unit to output the multi-spectral light of the set shape;
controlling, by the central controller, the image acquisition device to collect a fundus image under the environment of outputting the multi-spectral light of the set shape; and
processing, by the central controller, the fundus image to generate a final fundus image.

Preferably, when the multi-spectral light generating unit includes a first illumination unit and a second illumination unit, the fundus imaging system further includes an image acquisition device, and the photoconductive device connected to the first illumination unit and the second illumination unit includes a half-ring structure or two groups of quarter-ring structures, triggering, by the control unit, the specified light-emitting diode matrix of the specified illumination unit to emit the light with the preset wavelength and the preset energy; shaping, by the photoconductive device, the light emitted by the light-emitting diode matrix of the illumination unit to output the multi-spectral light of the set shape; where the step of controlling, by the central controller, the image acquisition device to collect and generate a fundus image under an environment of the multi-spectral light with the set shape includes:
controlling, by the control unit, the first illumination unit to turn on and emit light of a preset wavelength and preset energy, and the second illumination unit to turn off;
shaping, by the photoconductive device, the light emitted by the first illumination unit to output the multi-spectral light with the preset wavelength and the preset energy;
controlling, by the central controller, the image acquisition device to collect a first fundus image under the environment of the light of the preset wavelength and the preset energy;
controlling, by the control unit, the second illumination unit to turn on and emit light of a preset wavelength and preset energy, and the first illumination unit to turn off;
shaping, by the photoconductive device, the light emitted by the light-emitting diode matrix of the second illumination unit to output the multi-spectral light with the preset wavelength and the preset energy; and
controlling the image acquisition device to collect a second fundus image under the environment of the light of the preset wavelength.

Preferably, the step of processing, by the central controller, the fundus image to generate a final fundus image includes:
deleting, by the central controller, a corneal reflective area in the first fundus image;
acquiring, by the central controller, an effective image at a position in the second fundus image corresponding to the corneal reflective area; and
merging, by the control unit, the effective image into a corresponding position in the first fundus image to generate the final fundus image.

Preferably, the system further includes a light energy detector electrically connected to the control unit and mounted at one side of the light-emitting diode matrix, where the method further includes:
detecting, by the optical energy detector, energy of the multi-spectral light, and transmitting the energy of the multi-spectral light to the control unit; and
controlling, by the control unit, the light-emitting diode of the illumination unit to turn off when the energy of the multi-spectral light is greater than a preset threshold.

Preferably, the step of controlling, by the control unit, the first illumination unit to turn on and emit light of a preset wavelength and preset energy, and the second illumination unit to turn off includes:
controlling, by the control unit, multiple light-emitting diodes of the light-emitting diode matrix of the first illumination unit to emit light of a preset wavelength one by one in a predetermined sequence and a predetermined light emitting time, or, controlling, by the control unit, multiple light-emitting diodes of the light-emitting diode matrix of the first illumination unit to emit light simultaneously.

The embodiments of this application bring the following beneficial effects:
Embodiments of this application provides a multi-spectral generating unit, a fundus imaging system, and a fundus imaging method. The multi-spectral generating unit constitutes a multi-spectral light generating unit of the fundus imaging system. The unit includes one or more illumination units and a control unit. The illumination unit includes a light-emitting diode matrix, each light-emitting diode matrix is configured to emit light of multiple wavelengths. The control unit is configured to convert a control instruction sent from a central controller into a control signal to trigger a specified light-emitting diode matrix of a specified illumination unit to emit light of a preset wavelength and preset energy to output multi-spectral light. In this way, the multi-spectral generating unit with parameters that meets the user's needs such as shape, wavelength, energy, and flash time can be obtained. A multi-spectral generating unit with better performance is provided for the fundus imaging system or other imaging and illumination systems, which improves imaging ability to make imaging clearer. Furthermore, the photoconductive device can be set with different shapes and structures to output, so that the light of multi-spectral light has a set shape, such as full ring, half ring, quarter arc, etc. The central controller can control other parameters of the multi-spectral light through the control unit, so that the final output of the multi-spectral light can meet user requirements in terms of shape, numerical aperture, and luminous area.

Other features and advantages of this application will be explained in the subsequent description, or some features and advantages can be inferred from the description or unambiguously determined, or can be known by implementing the above technology of this application.

In order to make the above objects, features and advantages of this application more comprehensible, preferred embodiments are described below in conjunction with the accompanying drawings, which are described in detail below.

### BRIEF DESCRIPTION OF THE DRAWINGS

In order to more clearly explain the specific embodiments of this application or the technical solutions in the prior art, the following will briefly introduce the drawings required for the specific embodiments or the description of the prior art. Obviously, the drawings are some embodiments of this application. For those of ordinary skill in the art, without paying any creative labor, other drawings can also be obtained based on these drawings.
FIG. 1 is a schematic structural diagram of a multi-spectral light generating unit provided by an embodiment of this application;
FIG. 2 is a schematic structural diagram of another multi-spectral light generating unit provided by an embodiment of this application;FIG. 3 is a schematic structural diagram of still another multi-spectral light generating unit provided by an embodiment of this application;
FIG. 4 is a schematic structural diagram of an illumination unit in the multi-spectral light generating unit provided by an embodiment of this application;
FIG. 5 is a schematic structural diagram of a beam shaping unit and the illumination unit in the multi-spectral light generating unit provided by an embodiment of this application;
FIG. 6 is a schematic structural diagram of a square optical fiber bundle in the multi-spectral light generating unit provided by an embodiment of this application;
FIG. 7 is a schematic structural diagram of a further multi-spectral light generating unit provided by an embodiment of this application;
FIG. 8 is a schematic structural diagram of a further multi-spectral light generating unit provided by an embodiment of this application;
FIG. 9 is a schematic structural diagram of a further multi-spectral light generating unit provided by an embodiment of this application;
FIG. 10 is a multi-spectral retinal image generated by the multi-spectral light generating unit provided by an embodiment of this application;
FIG. 11 is a structural block diagram of a multi-spectral light generating system provided by an embodiment of this application;
FIG. 12 is a flowchart of a fundus imaging method provided by an embodiment of this application; and
FIG. 13 is a flowchart of specific sub-steps from step S1503 to step S1505 in FIG. 12.

Reference numerals: 100-multi-spectral light generating unit; 101-control unit; 102-illumination unit; 103-photoconductive device; 104-beam shaping unit; 105-central controller; 106-light energy detector; and 107-image acquisition device.

### DETAILED DESCRIPTION OF THE EMBODIMENTS

To make the objectives, technical solutions, and advantages of the embodiments of this application clearer, the technical solutions of this application will be described clearly and completely below with reference to the drawings. Obviously, the described embodiments are part of the embodiments of this application, but not all of the embodiments. Based on the embodiments in this application, all other embodiments obtained by those of ordinary skill in the art without making creative efforts fall within the protection scope of this application.

Considering that the existing light source configured for fundus imaging has poor performance, which results in the limitation of fundus imaging, the embodiments of this application provide a multi-spectral generating unit, a fundus imaging system, and a fundus imaging method. This technology can be applied in a fundus imaging system, and it can also be used in a fundus camera. In addition, the multi-spectral light generating unit can also be used in other imaging systems or illumination systems. This technology can be implemented by using related software or hardware, which will be described below by using embodiments.

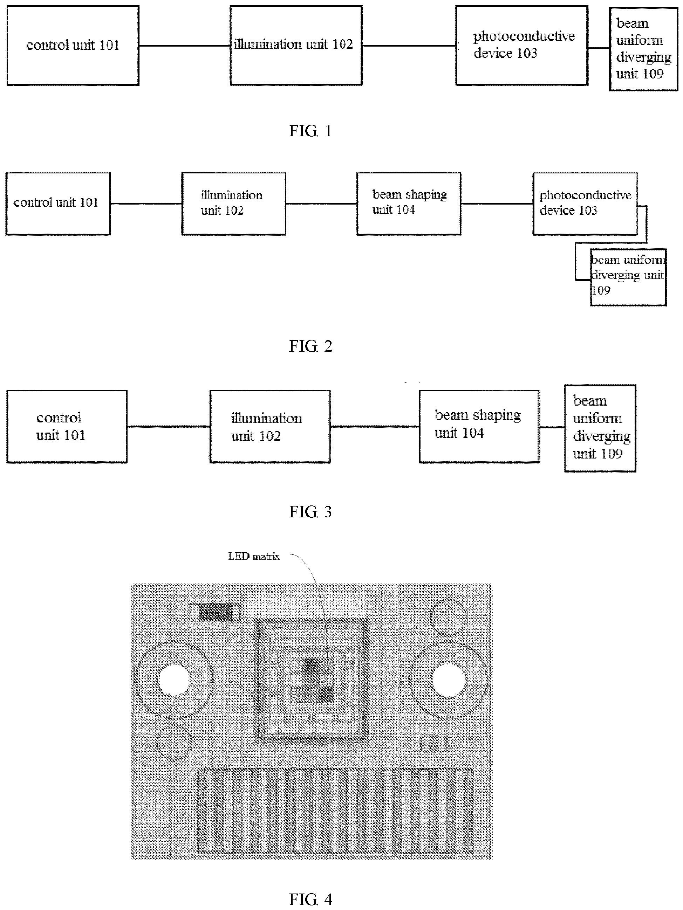

As shown in FIG. 1, FIG. 1 is a schematic structural diagram of a multi-spectral light generating unit 100. The multi-spectral light generating unit 100 includes a control unit 101, one or more illumination units 102, and a photoconductive device 103. Each illumination unit 102 includes a group of light-emitting diode matrices, and each group of light-emitting diode matrices may emit light of multiple wavelengths.

The control unit 101 converts a control instruction sent from the central controller 105 into a control signal to trigger the designated light-emitting diode matrix of the designated illumination unit 102 to emit light of a preset wavelength and preset energy. The photoconductive device 103 is configured to shape the light emitted by the light-emitting diode matrix of the illumination unit 102 to output multi-spectral light of a set shape.

In actual implementation, the above-mentioned control unit 101 may control the light-emitting diode matrix of the one or more illumination units 102 to emit light of a preset wavelength according to various light-emitting sequences. The photoconductive device 103 may be provided with different shapes and structures to output, so that the light of the multi-spectral light has a set shape, for example, a full ring shape, a half ring shape, a quarter arc shape, and so on. In addition, the multi-spectral light generating unit 100 may further include a beam uniform divergence unit 109 configured to further uniformly diverge and output the light of the set shape. The central controller 105 may control other parameters of the multi-spectral light through the control unit 101, so that the final output of the multi-spectral light can meet user requirements in terms of shape, numerical aperture, and luminous area.

The multi-spectral light generating unit 100 provided by an embodiment of this application includes one or more illumination units 102, a control unit 101, and a photoconductive device 103. The illumination unit 102 includes light-emitting diode matrices, each light-emitting diode matrix is configured to emit light of multiple wavelengths. The control unit 101 is configured to convert a control instruction sent from a central controller 105 into a control signal to trigger a specified light-emitting diode matrix of a specified illumination unit 102 to emit light of a preset wavelength and preset energy. The photoconductive device 103 is configured to shape the light emitted by the light-emitting diode matrix of the illumination unit 102 to output multi-spectral light of a set shape. In this way, the multi-spectral light with parameters that meets the user's needs such as shape, wavelength, energy, and flash time can be obtained. A multi-spectral generating unit 100 with better performance is provided for the fundus imaging system or other imaging and illumination systems, which improves imaging ability to make imaging clearer.

As shown in FIG. 2, FIG. 2 is a schematic structural diagram of another multi-spectral light generating unit 100. The multi-spectral light generating unit 100 includes a control unit 101, one or more illumination units 102, and a photoconductive device 103. Each illumination unit 102 includes light-emitting diode matrices, and each group of light-emitting diode matrices may emit light of multiple wavelengths. An input end of the beam shaping unit 104 is connected to the light-emitting diode matrix of the illumination unit 102. An output end of the beam shaping unit 104 is connected to the photoconductive device 103. The output end of the photoconductive device 103 is provided with a beam uniform divergence unit.

The control unit 101 may receive the control instruction sent by the central controller 105 to trigger the light-emitting diode matrix of the specified illumination unit 102 to emit light of a preset wavelength. The beam shaping unit 104 is configured to adjust distribution of light of each wavelength emitted by the light-emitting diode matrix of the illumination unit 102 to distribute the light of each wavelength uniformly on a cross section of the output end of the beam shaping unit 104. The photoconductive device 103 is configured to arrange optical fibers according to a set arrangement, and further shape output light of the beam shaping unit 104 to output the multi-spectral light with the set shape. Then the beam uniform divergence unit 109 further uniformly diverges and outputs the light of the set shape.

As shown in FIG. 3, FIG. 3 is a schematic structural diagram of still another multi-spectral light generating unit 100. The multi-spectral light generating unit 100 includes a control unit 101, one or more illumination units 102. The multi-spectral light generating unit 100 further includes a beam shaping unit 104.

An input end of the beam shaping unit 104 is connected to the light-emitting diode matrix of the illumination unit 102. The beam shaping unit 104 is configured to adjust distribution of light of each wavelength emitted by the light-emitting diode matrix of the illumination unit 102 to distribute the light of each wavelength uniformly on a cross section of the output end of the beam shaping unit 104. Then the beam uniform divergence unit 109 further uniformly diverges the light of the set shape to output the multi-spectral light of the set shape.

The light-emitting diode matrix of the above-mentioned illumination unit 102 may be formed by arranging multiple high-power light-emitting diodes. The light-emitting diode matrix of each group of illumination units 102 is arranged compactly. For example, if the illumination unit 102 includes a matrix of 9 light-emitting diodes, it may be arranged in the form of 3x3. If the illumination unit 102 includes a matrix of 16 light-emitting diodes, it may be arranged in the form of 4x4. Certainly, it may also include a matrix of a greater number of light-emitting diodes. The arranged light-emitting diodes of the illumination units 102 is usually rectangular, especially square. In this way, the multi-spectral light generating unit 100 may generate a light-emitting diode combination with a preset wavelength through spatial multiplexing.

In this embodiment, the beam shaping unit 104 may use a light homogenizing rod or other optical glass device to perform light homogenizing on the light of each wavelength emitted by the light-emitting diode matrix of the illumination unit 102, and then the processed light is transmitted to the photoconductive device 103. The photoconductive device 103 may also include multiple bundles of optical fibers, each bundle of optical fibers includes multiple cores, and the cores are regrouped and rearranged at the output end. The light of each wavelength emitted by the light-emitting diode matrix of the illumination unit 102 is subjected to light homogenizing processing, and then transmitted to the input end of the illumination light path.

The above-mentioned photoconductive device 103 may use a structural member or the like to rearrange the light beam, for example, in a semi-ring shape, a full ring shape, or the like.

In the multi-spectral light generating unit 100 provided by an embodiment of this application, the control unit 101 converts the control instruction sent from the central controller 105 into a control signal, and triggers the designated light-emitting diode matrix of the designated illumination unit 102 to emit light of a preset wavelength and preset energy. The beam shaping unit 104 is configured to adjust distribution of light of each wavelength emitted by the light-emitting diode matrix of the illumination unit 102 to distribute the light of each wavelength uniformly on a cross section of the output end of the beam shaping unit 104. The photoconductive device 103 is configured to arrange optical fibers arranged at the output end according to a set arrangement to output the multi-spectral light with a corresponding shape. In this way, the light of the light-emitting diode matrix may be subjected to light homogenizing processing, so that the light of various wavelengths may be uniformly irradiated on the fundus, which provides a multi-spectral light generating unit 100 with better performance to the fundus imaging system, improves the ability of fundus imaging, and makes the imaging clearer.

Generally, the light emitted by a light-emitting diode, laser, or other light source may be processed through light collection, multi-spectral channel aggregation, and multi-spectral optimization to obtain the desired cross-sectional shape and power of the optical path. Based on this, an embodiment of this application further provides a further multi-spectral light generating unit 100, which is implemented on the basis of the light source shown in FIG. 1. Specifically, as shown in FIG. 4, FIG. 4 is a schematic structural diagram of an illumination unit 102 in the multi-spectral light generating unit 100. The illumination unit 102 includes a matrix composed of a plurality of light-emitting diodes, and the light-emitting diode matrix is arranged in a square array. Certainly, the light-emitting diode matrix may also be arranged in other shapes, for example, directly arranged in a full ring shape or a semi-ring shape.

As shown in FIG. 4, the light-emitting diode matrix of the illumination unit 102 may be preferably implemented as follows. Each light-emitting diode is a square with a side length of 1 mm and has a characteristic quasi-Gaussian spectrum. When the matrix is composed of 9 light-emitting diodes, the matrix may be arranged in the form of 3x3. The 9 light-emitting diodes are compactly arranged, very close to each other, and the length of the square array formed may be 3.3 mm.

In actual implementation, in the matrix of 9 light-emitting diodes, each light-emitting diode corresponds to one wavelength, and there are a total of 9 wavelengths. It may also be 16 groups of light-emitting diode arrays arranged in the form of 4x4, corresponding to a total of 16 wavelengths. It may be 25 groups of light-emitting diode arrays arranged in the form of 5x5, corresponding to a total of 25 wavelengths.

In this embodiment, the above-mentioned photoconductive device 103 may be implemented in various forms. In one of the embodiments, the photoconductive device 103 includes optical fiber sub-bundles matching a number of the light-emitting diode matrix of the illumination unit 102. Each optical fiber sub-bundle of the photoconductive devices 103 includes a set number of optical fibers. At an input end of the photoconductive device 103, cores of each group of the optical fiber sub-bundles are uniformly arranged on a light-emitting surface of a corresponding light-emitting diode matrix. A position of each optical fiber sub-bundle is configured to correspond to a position of each light-emitting diode. Each optical fiber sub-bundle includes one or more optical fibers. Optical fibers with a set shape and a set number are formed at an output end of the photoconductive device 103. In addition, the optical fiber bundle may be realized by a combination of multiple step-index fibers.

Taking a 3x3 array of light-emitting diodes as an example for illustration, since the number of light-emitting diodes is 9, the photoconductive device 103 also includes 9 groups of fiber bundles. Each group of fiber bundles may include 25 optical fibers arranged as a square in form of 5x5. The diameter of each core is about 0.2 mm, then the side length of the square is about 1 mm, matching the side length of each light-emitting diode (1 mm). 9 sub-bundles are formed at an output end of the photoconductive device 103. Each sub-bundle includes 25 optical fibers, respectively from the above 9 groups of optical fiber bundles.

In the above manner, the light of each wavelength emitted by the light-emitting diode matrix of the illumination unit 102 may be uniformly distributed on the interface of the output end of the photoconductive device 103, and the uniformity quantization interval is related to the diameter of the core in each group of fiber bundles. Certainly, it is also possible to use a core with a smaller diameter so that each group of fiber bundles may includes more cores under the same area to reduce the above-mentioned quantization interval and improve the uniformity of the spectrum.

The above-mentioned sub-bundles may be arranged in any shape according to actual imaging requirements, for example, arranged in a ring shape, a semi-ring shape or other partial ring shapes.

In this embodiment, the above-mentioned beam uniform divergence unit 109 may be a frosted glass diffuser, which is simple, low-cost, and achieves a good astigmatism effect.

Further, the above mentioned multi-spectral light generating unit 100 further includes a light energy detector 106. The light energy detector 106 may be directly mounted near the light-emitting diode matrix, or may be connected to one or more fiber cores in the optical fiber bundle, and configured to detect output power and energy of the light source. After being calibrated, the optical power detector may perform real-time power monitoring, electronic control and safe hard cutoff. One of the above mentioned sub-bundle is connected to a photodetector or an optical power detector, and is configured for power monitoring. The detected power parameters may be fed back to the control unit 101, so that the user may effectively control and adjust the luminous power and energy flexibly, thereby ensuring that the daily exposure is below the safety threshold level to avoid damage to eyes caused by light.

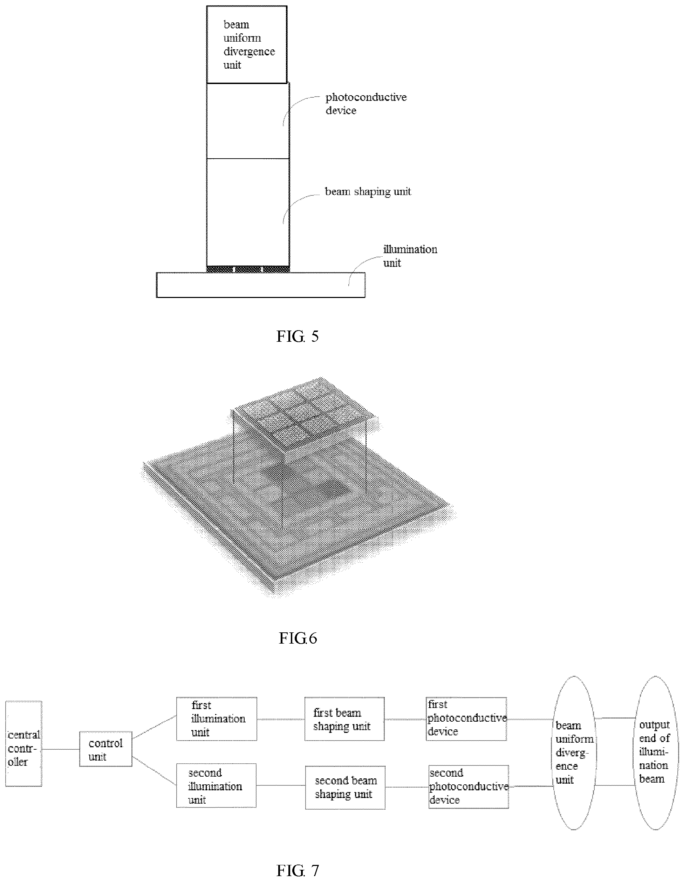

In another embodiment, as shown in FIG. 5, FIG. 5 is a schematic structural diagram of a beam shaping unit 104 and the illumination unit 102 in the multi-spectral light generating unit 100. The beam shaping unit 104 includes a light homogenizing device. The light homogenizing device may specifically be a light homogenizing rod. An input end of the light homogenizing rod is oppositely coupled to the light-emitting diode matrix of the illumination unit 102. A shape of the input end of the light homogenizing rod is configured to match a shape of the light-emitting diode matrix. An output end of the light homogenizing rod is docked with an optical fiber bundle of the photoconductive device 103.

If the light homogenizing rod is long enough, the light from each light-emitting diode will be fully occupied in space before reaching the output end of the light homogenizing rod. The length of the light homogenizing rod may be 15 mm to 40 mm. The output end of the light homogenizing rod is docked with multiple optical fibers that may be arranged to form any desired shape. In this embodiment, the optical fiber bundle may be set as a square optical fiber bundle. As shown in FIG. 6, FIG. 6 is a schematic structural diagram of a square optical fiber bundle in the multi-spectral light generating unit 100. The square optical fiber bundle has a square end portion, which may improve the optical coupling efficiency of the optical system.

In one of the modes, the light homogenizing rod is a square glass light homogenizing rod, the end face of the input end of the light homogenizing rod has a length of 3.4 mm, so as to cover the light-emitting diode matrix of the illumination unit 102 in the form of 3x3 (with a side length being 3.3 mm). The ring diameter at the output end of the light homogenizing rod may be 3.4 mm, so as to match the square input end of the half-ring optical fiber bundle of 216 fibers. The length of the light homogenizing rod may be 20 mm. The coupling efficiency of the light homogenizing rod in an optical system with a numerical aperture of 0.28 is 4%, and the spiral conduction mode may be avoided.

In another mode, the above-mentioned light homogenizing rod may be matched with 9 optical fiber bundles. The length of the end face of the input end of the light homogenizing rod is 3.6 mm, and the width of the output end is 3.6 mm. In this case, each of the 9 optical fiber bundles includes 12 cores. The length of the light homogenizing rod may be 20 mm. The coupling efficiency of the light homogenizing rod in an optical system with a numerical aperture of 0.28 is 1.57%, and the spiral conduction mode may be avoided.

The above-mentioned light homogenizing rod may be directly connected to the light emitting surface of the light-emitting diode matrix of the illumination unit 102, so that light of any wavelength emitted from the light-emitting diode matrix may be uniformly projected into the optical fiber. The light homogenizing rod may has a square cross section, or in a three-dimensional conical shape. Illumination is output according to the waveguide characteristics of the rod.

As shown in FIG. 7, FIG. 7 is a schematic structural diagram of a further multi-spectral light generating unit 100. The multi-spectral light generating unit 100 includes one or two illumination units 102, one or two beam shaping units 104 corresponding to a number of the illumination units 102, and one or two photoconductive devices 103. The control unit 101 is configured to convert the control instruction sent from the central controller into the control signal to control the one or two illumination units 102. The light-emitting diodes of each illumination unit 102 are directly controlled by the control unit 101 respectively. In this embodiment, the multi-spectral light generating unit 100 includes a first illumination unit, a second illumination unit, a first beam shaping unit, a second beam shaping unit, a first photoconductive device, and a second photoconductive device.

The distribution of the multi-spectral light generating unit 100 needs to ensure that light sources of various wavelengths may uniformly illuminate the fundus, and ensure that the cross section of the entrance optical path and the cross section of the exit optical path at the pupil of the eye are kept at a certain distance to overcome the unnecessary interference of fundus backscatter in the image. Generally, illumination is provided by a ring/arc, while light is reflected through the fundus of the central circular area for image acquisition.

In the image acquisition device 107, the ring illumination is usually divided into partial rings or arcs, which only illuminates part of the ring at a single time. Generally, it is necessary to quickly turn on two partial ring illuminations to acquire two separate images and merge them into a complete image with a desired field angle. The individual images of this sequential ring shooting technology usually have a certain area of corneal reflection, but when the two separate images are merged, the corneal reflection may be effectively eliminated. In this electronic image overlay, the pixel gray value of the darker area of the image acquired by a single half-ring illumination will double. To prevent any overlap of the images, the angle of the partial ring illumination should be less than 180 degrees. When the image is repaired, there may be no obvious dividing line, and a gradual transition between the two images may be achieved.

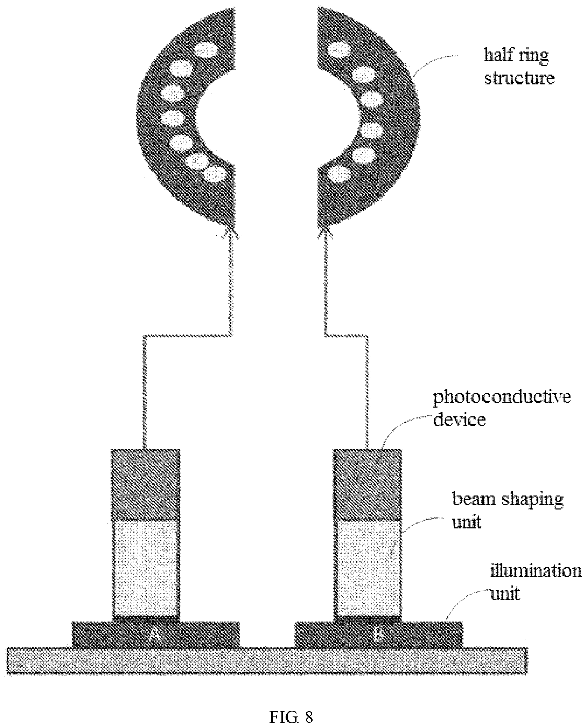

Based on this, in one of the embodiments, as shown in FIG. 8, FIG. 8 is a schematic structural diagram of a further multi-spectral light generating unit 100. The output end of the photoconductive device 103 is a semi-ring structure. The semi-ring structure is realized by arranging the optical fibers into a semi-ring. The half-ring structures of the output ends of the two photoconductive devices 103 are configured to match each other. The half-ring structures of the output ends of the two photoconductive devices 103 are configured to be combined to form a full ring structure.

Specifically, the two half-ring structures are separated from each other, and each half-ring structure corresponds to an illumination unit 102. When the control unit 101 sends a control signal and a control current to a light-emitting diode of a certain wavelength on the specific illumination unit 102, the corresponding illumination may be through the above light homogenizing rod and optical fibers, causing the semi-ring structure to emit light.

Through the above two half-ring structures separated from each other, two arc-shaped illumination areas may be formed. The two arc-shaped illumination areas are uniformly distributed along a same circumference. The two arc-shaped illumination areas are configured to illuminate a same part of the patient's fundus, and central angles corresponding to the two arc-shaped illumination areas are both less than 180 degrees.

Each arc-shaped illumination area is derived from the corresponding semicircular structure in the multi-spectral light generating unit 100, and the image is nearly semicircular on the pupil of the eye to be measured, thereby further illuminating the entire retina within a predetermined solid angle (field of view).

As another embodiment, the output end of the photoconductive device 103 includes two groups of quarter ring structures. Each quarter ring structure is configured to arrange the optical fibers at the output end of the photoconductive device 103 in an arc shape. The quarter ring structures are uniformly distributed along a same circumference at the output ends of the two photoconductive devices 103. The four quarter ring structures are uniformly distributed along a same circumference to form four arc-shaped illumination areas. The four arc-shaped illumination areas are configured to illuminate a same part of the patient's fundus, and the center angles corresponding to the four arc-shaped illumination areas are all less than 90 degrees.

In the four-arc illumination mode, the illumination may be provided by two corresponding illumination units 102 of the multi-spectral light generating unit 100, respectively. Each corresponding photoconductive device 103 may divide the output light into two parts, and provide them to the two opposite but not adjacent ring-shaped illumination areas.

Further, the four-light ring light beam provided by the multi-spectral light generating unit 100 may be directly brought to the input end of the illumination light path, and each light ring is nearly a quarter ring. It is imaged on the pupil of the eye to be measured through the illumination optical path. Thereby further illuminating the entire retina within a predetermined solid angle (field of view) through the illumination optical path.

Each illumination unit 102 may illuminate two opposing quarter rings. In actual implementation, each two of the four quarter ring structures alternately emit light. Specifically, after one of the illumination units 102 of the multi-spectral light generating unit 100 (for example, responsible for the two opposing quarter ring structures) emits illumination light of a wavelength 1, the illumination unit 102 corresponding to the other branch (for example, responsible for the other two opposing quarter ring structures) then emits illumination light of the wavelength 1. Then, after the first branch emits illumination light of a wavelength 2, the second branch also emits illumination light of the wavelength 2, successively, and so on. While the arc-shaped illumination area on each side is illuminated, the image acquisition device 107 simultaneously performs image acquisition.

Certainly, one of the illumination units 102 may be responsible for two adjacent quarter ring structures, and the other illumination unit 102 may be responsible for the other two adjacent quarter ring structures.

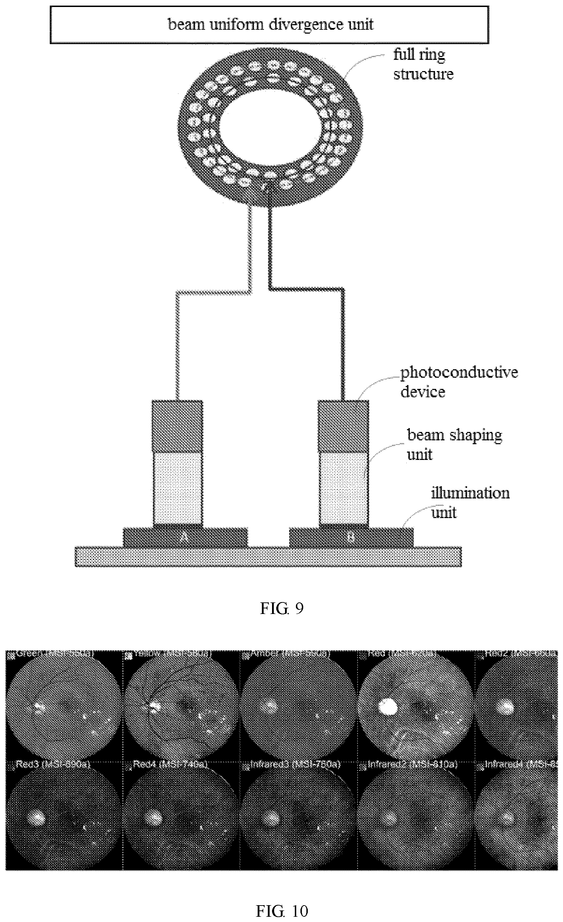

In another embodiment, as shown in FIG. 9, FIG. 9 is a schematic structural diagram of a further multi-spectral light generating unit 100. Each output end of the photoconductive device 103 includes a full ring structure. The full ring structure is configured to arrange the optical fibers at the output end of the beam shaping unit 104 into a full ring with a set radius. One full ring of the two output ends of the two photoconductive devices 103 is an outer ring structure, and the other full ring of the two output ends of the two photoconductive devices 103 is an inner ring structure, where a radius of the outer ring structure is larger than a radius of the inner ring structure.

Specifically, the optical fibers are directly transmitted to the illumination optical path through the set optical fiber output ends arranged into two full rings inside and outside, and its lens system may merge the light into a complete ring.

In this way, half-ring illumination, quarter-ring illumination, and full-ring illumination may be achieved. In half-ring illumination, the light-emitting diodes may include 9 wavelengths. In full-ring illumination, the light-emitting diodes may include up to 18 wavelengths. As shown in FIG. 10, FIG. 10 is a multi-spectral retinal image generated by the multi-spectral light generating unit 100. FIG. 10 shows the retinal images under 10 kinds of spectrums, and the retinal images under each spectrum shows different characteristics.

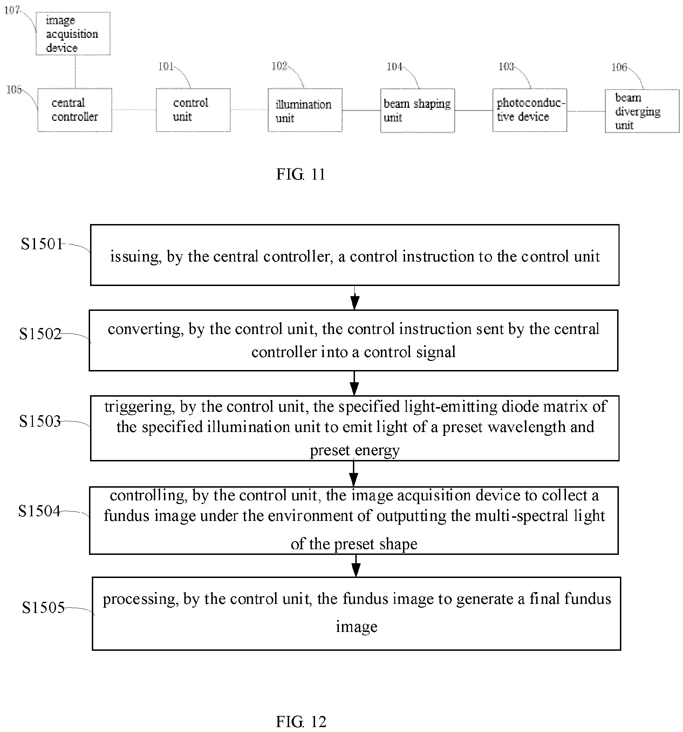

Corresponding to the above embodiment of the multi-spectral light generating unit 100, as shown in FIG. 11, FIG. 11 is a structural block diagram of a fundus imaging system. The system includes the multi-spectral light generating unit 100 described in the above embodiments, and it also includes a central controller 105 and an image acquisition device 107, and the central controller 105 and the image acquisition device 107 are in communication connection with the control unit 101, respectively.

The central controller 105 is configured to issue a control instruction to the multi-spectral light generating unit 100.

The multi-spectral light generating unit 100 is configured to convert a control instruction sent from a central controller 105 into a control signal to trigger a specified light-emitting diode matrix of a specified illumination unit 102 to emit light of a preset wavelength and preset energy to output multi-spectral light.

The image acquisition device 107 is configured to collect a fundus image under an environment of the multi-spectral light.

The system further includes a light energy detector 106 electrically connected to the control unit 101.

The light energy detector 106 is mounted at one side of the light-emitting diode matrix, and configured to detect energy of the multi-spectral light and transmit the energy of the multi-spectral light to the control unit 101.

The control unit 101 is configured to control the light-emitting diode of the illumination unit 102 to turn off when the energy of the multi-spectral light is greater than a preset threshold.

When the multi-spectral light generating unit 100 of the fundus imaging system includes two illumination units 102, the control unit 101 may send control signals to the two illumination units 102 (e.g., unit a and unit b) respectively, where the two illumination units 102 are independently of each other. The control unit 101 may also control each light-emitting diode in any illumination unit 102 to turn on or off in any sequence. In addition, each light-emitting diode on unit a may be combined with any light-emitting diode on unit b, where the unit a and the unit b may have a same wavelength or completely different wavelengths.

The fundus imaging system provided by an embodiment of this application has the same technical characteristics as the multi-spectral light generating unit 100 provided by the above embodiment, so it may also solve the same technical problems and achieve the same technical effect.

As shown in FIG. 12, corresponding to the above embodiment of the multi-spectral light generating unit 100, an embodiment of this application further provides a fundus imaging system, which includes the multi-spectral light generating unit 100 of any of the above embodiments . It should be noted that the fundus imaging system provided in this embodiment has the same basic principles and technical effects as the corresponding embodiments. For brief description, the parts not mentioned in this embodiment may be refered to the corresponding content in the above embodiments. The fundus imaging system further includes a central controller 105 and an image acquisition device 107, and the central controller 105 and the image acquisition device 107 are in communication connection with the control unit 101, respectively.

The central controller 105 is configured to issue a control instruction to the multi-spectral light generating unit 100.

The multi-spectral light generating unit 100 is configured to convert a control instruction sent from a central controller 105 into a control signal to trigger a specified light-emitting diode matrix of a specified illumination unit 102 to emit light of a preset wavelength and preset energy to output multi-spectral light.

The image acquisition device 107 is configured to collect a fundus image under an environment of the multi-spectral light.

In addition, the system further includes a light energy detector 106 electrically connected to the control unit 101.

The light energy detector 106 is mounted at one side of the light-emitting diode matrix, and configured to detect energy of the multi-spectral light and transmit the energy of the multi-spectral light to the control unit 101.

The control unit 101 is configured to control the light-emitting diode of the illumination unit 102 to turn off when the energy of the multi-spectral light is greater than a preset threshold.

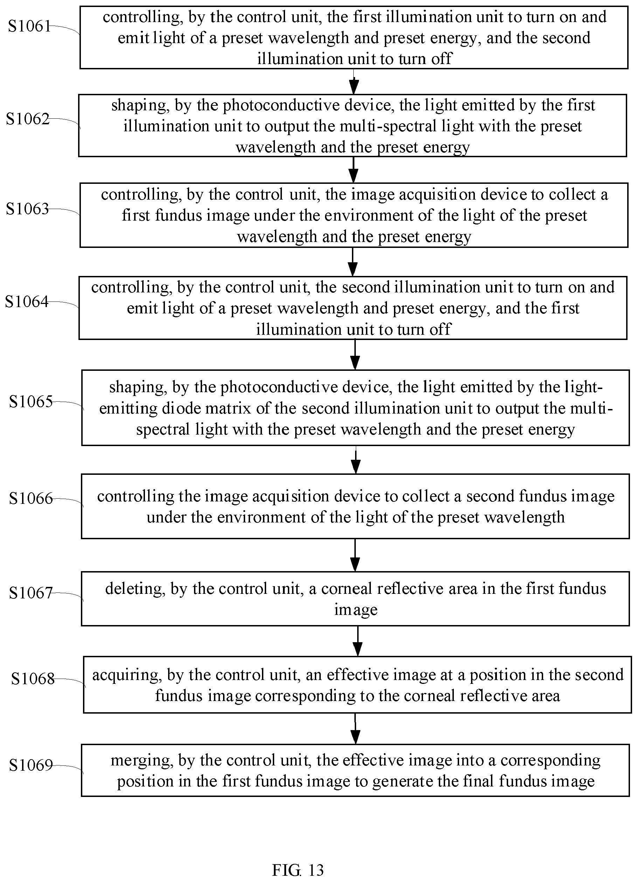

As shown in FIG. 13, corresponding to the above-mentioned multi-spectral light generating unit 100 embodiment, an embodiment of this application further provides a fundus imaging method, which is applied to a fundus imaging system including a central controller 105, an image acquisition device 107, and the above-described multi-spectral light generating unit 100. The central controller 105 and the image acquisition device 107 are in communication connection with the control unit 101, respectively, where the method includes:
Step S1501: issuing, by the central controller 105, a control instruction to the control unit 101;
Step S1502: converting, by the control unit 101, the control instruction sent by the central controller 105 into a control signal; and
Step S1503: triggering, by the control unit 101, the specified light-emitting diode matrix of the specified illumination unit 102 to emit light of a preset wavelength and preset energy.

Specifically, multiple light-emitting diodes of the light-emitting diode matrix of the first illumination unit are controlled by the control unit 101 to emit light of a preset wavelength one by one in a predetermined sequence and a predetermined light emitting time, or, multiple light-emitting diodes of the light-emitting diode matrix of the first illumination unit are controlled by the control unit 101 to emit light simultaneously. In addition, a series of images corresponding to each light source one-by-one may be collected by the image acquisition device 107 in synchronization with the light-emitting diode flash, and the collected eyeball images may be transmitted to an external computing device through the central controller 105 to be configured to be stored, read or further processed. In normal applications, the eyeball images illuminated by the spectrum of each light-emitting diode are collected separately.

When the multi-spectral light generating unit 100 receives a light emitting instruction or light emitting trigger pulse (ie, a control instruction) from the central controller 105, the light-emitting diode matrix of the illumination unit 102 associated with the light emitting instruction or light emitting trigger pulse emits light according to the specified flash time. The controller 105 may simultaneously send signals to the multi-spectral light source and the image acquisition device 107, or the multi-spectral light source simultaneously sends signals to the image acquisition device 107 immediately after starting to emit light, so that the image acquisition device 107 and the multi-spectral light source may work synchronously.
Step S1504: shaping, by the photoconductive device 103, the light emitted from the light-emitting diode matrix of the illumination unit 102 to output the multi-spectral light of the set shape;
Step S1505: controlling, by the central controller 105, the image acquisition device 107 to collect a fundus image under the environment of outputting the multi-spectral light of the set shape; and
Step S1506: processing, by the central controller 105, the fundus image to generate a final fundus image.

Specifically, in this embodiment, when the multi-spectral light generating unit 100 includes a first illumination unit and a second illumination unit, the fundus imaging system further includes an image acquisition device 107, and a photoconductive device 103 to which the first illumination unit and the second illumination unit are connected includes a half-ring structure or two groups of quarter-ring structures, the steps S1503 to S1505 may be specifically performed by sub-steps as follows.
Step S1601: controlling, by the control unit 101, the first illumination unit to turn on and emit light of a preset wavelength and preset energy, and the second illumination unit to turn off;
Step S1602: shaping, by the photoconductive device 103, the light emitted by the first illumination unit to output the multi-spectral light with the preset wavelength and the preset energy;
Step S1603: synchronizing the control unit 101 with the image acquisition device 107 and collecting a first fundus image under the environment of the light of the preset wavelength and the preset energy;
Step S1604: controlling, by the control unit 101, the second illumination unit to turn on and emit light of a preset wavelength and preset energy, and the first illumination unit to turn off;
Step S1605: shaping, by the photoconductive device 103, the light emitted by the light-emitting diode matrix of the second illumination unit to output the multi-spectral light with the preset wavelength and the preset energy; and
Step S1606: controlling, by the central controller 105, the image acquisition device 107 to collect a second fundus image under the environment of the light of the preset wavelength.
Step S1506 may be performed by sub-steps as follows.
Step S1607: deleting, by the central controller 105, a corneal reflective area in the first fundus image;
Step S1608: acquiring, by the central controller 105, an effective image at a position in the second fundus image corresponding to the corneal reflective area; and
Step S1609: merging, by the control unit 105, the effective image into a corresponding position in the first fundus image to generate the final fundus image.

In addition, the system further includes a light energy detector 106 electrically connected to the control unit 101 and mounted at one side of the light-emitting diode matrix, where the method further includes:
Step S1507: detecting, by the optical energy detector 106, energy of the multi-spectral light, and transmitting the energy of the multi-spectral light to the control unit 101; and
Step S1508: controlling, by the control unit 101, the light-emitting diode of the illumination unit 102 to turn off when the energy of the multi-spectral light is greater than a preset threshold.

The output optical power and energy of the light-emitting diode matrix of each illumination unit 102 are calibrated and real-time monitored to realize real-time monitoring of the energy of each light-emitting diode each time it emits light, and during normal or abnormal operation, once the energy reaches the safe energy warning limit (that is, the preset threshold value), cut off the light source immediately to ensure absolute safety protection for the patient.

In this way, an effective image may be used to replace the corneal reflective area, which makes the fundus image clearer and more accurate, and provides more fundus data for the user.

In the multi-spectral light generating unit, the fundus imaging system, and the fundus imaging method provided by the embodiments of this application, the multi-spectral light generating unit 100 is a multi-spectral illumination source with a series of modes. The series of modes may briefly trigger each wavelength light source in sequence, multiple images (multiple wavelength images) may be captured within a series, and the series period is usually less than 250 milliseconds. In the series, different wavelengths may be combined or a single wavelength may be repeated or different wavelength may flash in sequence. Wide field angle fundus illumination may be achieved by ring-shaped multi-spectral illumination. The multi-spectral light generating unit 100 may meet the multiple requirements required for multi-spectral fundus imaging, and the optional light-emitting diodes required for clinical applications may provide sufficient power to meet the illumination requirements for clear fundus imaging.

In the multi-spectral light generating unit, the fundus imaging system, and the fundus imaging method provided by the embodiments of this application, a single source may be divided into multiple narrow-band spectra (with a bandwidth of less than 30 nanometers), or multiple narrow-band spectra may be integrated into a single light source to acquire retinal imaging capabilities. The illumination power may be effectively controlled without causing light damage, and the daily exposure is far below the safety threshold level. The multi-spectral light generating unit 100 is a wavelength-based modular design, which may be flexibly selected according to actual needs. The amount of light given may be dynamically adjusted based on the timely power monitoring of the light source and the measured feedback results of the retinal reflective characteristics.

The computer program product of the multi-spectral light generating unit, the fundus imaging system, and the fundus imaging method provided by the embodiments of this application includes a computer-readable storage medium storing program code, and the instructions included in the program code may be configured to perform the method described in the foregoing embodiments, and specific implementation may be refered to the method embodiments, and details are not described herein again.

In addition, in the description of the embodiments of this application, it should be noted that the terms "installation", "link", and "connection" should be understood in a broad sense, for example, it may be a fixed connection or a removable connection, or a integral connection; it may be either mechanical connection or electrical connection; it may be direct connection, or indirect connection through an intermediary, or internal connection between two components. For those of ordinary skill in the art, the specific meaning of the above terms in the present disclosure may be understood in specific situations.

If the function is implemented in the form of a software functional unit and sold or used as an independent product, it can be stored in a readable storage medium on a computing platform. Based on such an understanding, the technical solution of this application essentially or part of the contribution to the existing technology or part of the technical solution can be embodied in the form of a software product, the computer software product is stored in a storage medium, including several instructions to make a computer unit (which may be an embedded central processing unit, an image processing unit, a personal computer, a server, or a network device, etc.) execute all or part of the steps of the methods described in the embodiments of this application. The aforementioned storage media include: U disk, mobile hard disk, ROM (Read-Only Memory), RAM (Random Access Memory), magnetic disk or optical disk and other media that can store program codes.

In the description of this application, it should be noted that the terms "center", "upper", "lower", "left", "right", "vertical", "horizontal", "inner", "outer", etc. indicate the orientation or positional relationship based on the orientation or positional relationship shown in the drawings, only for the convenience of describing the present application and simplify the description, rather than indicating or implying that the device or element referred to must have a specific orientation, be constructed and operate in a specific orientation, and therefore cannot be construed as limiting this application. In addition, the terms "first", "second", and "third" are for descriptive purposes only, and cannot be understood as indicating or implying relative importance.

Finally, it should be noted that the above-mentioned embodiments are only specific implementations of this application, which are used to illustrate the technical solutions of this application, rather than limit them, and the scope of this application is not limited thereto. Although the present disclosure has been described in detail with reference to the foregoing embodiments, those of ordinary skill in the art should understand that: any person skilled in the art can still modify the technical solutions described in the foregoing embodiments within the scope disclosed in this application or it is easy to think of changes, or equivalent replacement of some of the technical features. These modifications, changes, or replacements do not deviate from the spirit and scope of the technical solutions of the embodiments of this application, and should be covered within the scope of this application. Therefore, the scope of this application shall be subject to the scope of the claims.

### INDUSTRIAL APPLICABILITY

The multi-spectral generating unit, the fundus imaging system, and the fundus imaging method may provides the multi-spectral generating unit that meets the user's needs such as shape, wavelength, energy, and flash time, which provides a multi-spectral generating unit with better performance for the fundus imaging system or other imaging and illumination systems, which improves imaging ability to make imaging clearer. The final output multi-spectral light may meet the user's needs in terms of shape, numerical aperture and luminous area.

## Claims

1. A multi-spectral light generating unit, **characterized by** comprising a control unit and one or more illumination units; each illumination unit comprises a light-emitting diode matrix, each light-emitting diode matrix is configured to emit light of multiple wavelengths;
the control unit is configured to convert a control instruction sent from a central controller into a control signal to trigger a specified light-emitting diode matrix of a specified illumination unit to emit light of a preset wavelength and preset energy to output multi-spectral light;
the multi-spectral light generating unit further comprises a photoconductive device connected to the one or more illumination units; and
the photoconductive device is configured to shape the light emitted from the light-emitting diode matrix of the illumination unit to output multi-spectral light with a set shape.

2. The multi-spectral light generating unit according to claim 1, **characterized by** further comprising a beam shaping unit;
an input end of the beam shaping unit is connected to the light-emitting diode matrix of the illumination unit; an output end of the beam shaping unit is connected to the photoconductive device; and the beam shaping unit is configured to adjust distribution of light of each wavelength emitted by the light-emitting diode matrix of the illumination unit to distribute the light of each wavelength uniformly on a cross section of the output end of the beam shaping unit; and
the photoconductive device is configured to arrange optical fibers according to a set arrangement, and further configured to shape output light of the beam shaping unit to output the multi-spectral light generating unit with the set shape.

3. The multi-spectral light generating unit according to claim 1 or 2, **characterized by** further comprising a beam uniform divergence unit mounted at an end of the photoconductive device to further spread out light of a light beam with a set shape uniformly.

4. The multi-spectral light generating unit according to claim 1 or 2, **characterized in that** the photoconductive device comprises optical fiber sub-bundles matching a number of the light-emitting diode matrices of the illumination unit; each optical fiber sub-bundle of the photoconductive devices comprises a set number of optical fibers; and
at an input end of the photoconductive device, cores of each group of the optical fiber sub-bundles are uniformly arranged on a light-emitting surface of a corresponding light-emitting diode matrix; a position of each optical fiber sub-bundle is configured to correspond to a position of each light-emitting diode; each optical fiber sub-bundle comprises one or more optical fibers; and optical fibers with a set shape and a set number are formed at an output end of the photoconductive device.

5. The multi-spectral light generating unit according to claim 2, **characterized in that** the beam shaping unit is a light homogenizing rod; an input end of the light homogenizing rod is oppositely coupled to the light-emitting diode matrix of the illumination unit; a shape of the input end of a light homogenizing device is configured to match a shape of the light-emitting diode matrix of the illumination unit; and an output end of the light homogenizing device is docked with an optical fiber bundle of the photoconductive device.

6. The multi-spectral light generating unit according to claim 3, **characterized in that** the beam uniform divergence unit is a frosted glass diffuser.

7. The multi-spectral light generating unit according to claim 2 or 5 or 6, **characterized by** comprising one or two illumination units, one or two beam shaping units corresponding to a number of the illumination units, and one or two photoconductive devices; and
the control unit is configured to convert the control instruction sent from the central controller into the control signal to control the one or two illumination units.

8. The multi-spectral light generating unit according to claim 7, **characterized by** comprising two illumination units, two beam shaping units, and two photoconductive devices, and an output end of each photoconductive device has a half-ring structure; and
the half-ring structures of the output ends of the two photoconductive devices are configured to match each other; and the half-ring structures of the output ends of the two photoconductive devices are configured to be combined to form a full ring structure.

9. The multi-spectral light generating unit according to claim 8, **characterized in that** the output end of the photoconductive device comprises a full ring structure;
the full ring structure is configured to arrange the optical fibers at the output end of the beam shaping unit into a full ring with a set radius; one full ring of the two output ends of the two photoconductive devices is an outer ring structure, and the other full ring of the two output ends of the two photoconductive devices is an inner ring structure, wherein a radius of the outer ring structure is larger than a radius of the inner ring structure.

10. The multi-spectral light generating unit according to claim 8, **characterized in that** the output end of the photoconductive device comprises two groups of quarter ring structures; and
each quarter ring structure is configured to arrange the optical fibers at the output end of the photoconductive device in an arc shape; and the quarter ring structures are uniformly distributed along a same circumference at the output ends of the two photoconductive devices.

11. The multi-spectral light generating unit according to claim 1, **characterized by** further comprising a beam shaping unit; and
an input end of the beam shaping unit is connected to the light-emitting diode matrix of the illumination unit; the beam shaping unit is configured to adjust distribution of light of each wavelength emitted by the light-emitting diode matrix of the illumination unit to distribute the light of each wavelength uniformly on a cross section of the output end of the beam shaping unit to output multi-spectral light with a set shape.

12. The multi-spectral light generating unit according to any one of claims 1 to 11, **characterized in that** the light-emitting diode matrix of the illumination unit is arranged in a square array.

13. A fundus imaging system, **characterized by** comprising the multi-spectral light generating unit according to any one of claims 1 to 12; the fundus imaging system further comprises a central controller and an image acquisition device, the central controller and the image acquisition device are in communication connection with the control unit, respectively;
the central controller is configured to issue a control instruction to the multi-spectral light generating unit;
the multi-spectral light generating unit is configured to convert the control instruction sent from the central controller into a control signal to trigger a specified light-emitting diode matrix of a specified illumination unit to emit light of a preset wavelength and preset energy to output multi-spectral light; and
the image acquisition device is configured to collect a fundus image under a multi-spectral light environment.

14. The fundus imaging system according to claim 13, **characterized by** further comprising a light energy detector; the light energy detector is electrically connected to the control unit;
the light energy detector is mounted at one side of the light-emitting diode matrix, and configured to detect energy of the multi-spectral light, and transmit the energy of the multi-spectral light to the control unit; and
the control unit is configured to control the light-emitting diode of the illumination unit to turn off when the energy of the multi-spectral light is greater than a preset threshold.

15. A fundus imaging method, **characterized in that** the method is applied to a fundus imaging system comprising a central controller, an image acquisition device, and the multi-spectral light generating unit according to any one of claims 1 to 7; and the central controller and the image acquisition device are in communication connection with the control unit, respectively, wherein the method comprises:
issuing, by the central controller, a control instruction to the control unit;
converting, by the control unit, the control instruction sent by the central controller into a control signal;
triggering, by the control unit, a specified light-emitting diode matrix of a specified illumination unit to emit light of a preset wavelength and preset energy;
shaping, by the photoconductive device, the light emitted from the light-emitting diode matrix of the illumination unit to output multi-spectral light of a set shape;
controlling, by the central controller, the image acquisition device to collect a fundus image under an environment of the multi-spectral light with the set shape; and
processing, by the central controller, the fundus image to generate a final fundus image.

16. The method according to claim 15, **characterized in that** when the multi-spectral light generating unit comprises a first illumination unit and a second illumination unit, the fundus imaging system further comprises an image acquisition device, and the photoconductive device connected to the first illumination unit and the second illumination unit comprises a half-ring structure or two groups of quarter-ring structures, triggering, by the control unit, the specified light-emitting diode matrix of the specified illumination unit to emit the light with the preset wavelength and the preset energy; shaping, by the photoconductive device, the light emitted by the light-emitting diode matrix of the illumination unit to output the multi-spectral light of the set shape; wherein the step of controlling, by the central controller, the image acquisition device to collect and generate a fundus image under an environment of the multi-spectral light with the set shape comprises:
controlling, by the central controller, the first illumination unit to turn on and emit the light of the preset wavelength and the preset energy, and the second illumination unit to turn off;
shaping, by the photoconductive device, the light emitted by the first illumination unit to output the multi-spectral light of the preset wavelength and the preset energy;
controlling, by the central controller, the image acquisition device to collect a first fundus image under the environment with the light of the preset wavelength and the preset energy;
controlling, by the central controller, the second illumination unit to turn on and emit the light of the preset wavelength and the preset energy, and the first illumination unit to turn off;
shaping, by the photoconductive device, the light emitted by the light-emitting diode matrix of the second illumination unit to output the multi-spectral light with the preset wavelength and the preset energy; and
controlling the image acquisition device to collect a second fundus image under the environment with the light of the preset wavelength.

17. The method according to claim 15 or 16, **characterized in that** the step of processing, by the central controller, the fundus image to generate a final fundus image comprises:
deleting, by the control unit, a corneal reflective area in the first fundus image;
acquiring, by the control unit, an effective image at a position in the second fundus image corresponding to the corneal reflective area; and
merging, by the control unit, the effective image into a corresponding position in the first fundus image to generate the final fundus image.

18. The method according to any one of claims 17 to 18, **characterized in that** the system further comprises a light energy detector electrically connected to the control unit and mounted at one side of the light-emitting diode matrix, wherein the method further comprises:
detecting, by the optical energy detector, energy of the multi-spectral light, and transmitting the energy of the multi-spectral light to the control unit; and
controlling, by the control unit, the light-emitting diode of the illumination unit to turn off when the energy of the multi-spectral light is greater than a preset threshold.

19. The method according to any one of claims 14 to 18, **characterized in that** the step of controlling, by the central controller, the first illumination unit to turn on and emit light of the preset wavelength and the preset energy, and the second illumination unit to turn off comprises:
controlling, by the control unit, multiple light-emitting diodes of the light-emitting diode matrix of the first illumination unit to emit light of a preset wavelength one by one in a predetermined sequence and a predetermined light emitting time, or, controlling, by the control unit, multiple light-emitting diodes of the light-emitting diode matrix of the first illumination unit to emit light simultaneously.
